# EUROPEAN PATENT SPECIFICATION

(11) **EP 1 691 688 B1**
(45) Date of publication and mention of the grant of the patent: **20.08.2008**
(21) Application number: 04801431.0
(22) Date of filing: 01.12.2004
(51) Int. Cl.: A61B 8/00, G01N 29/24

(54) **ERGONOMIC HOUSING FOR ULTRASOUND TRANSDUCER PROBE**
ERGONOMISCHES GEHÄUSE FÜR EINE ULTRASCHALLWANDLERSONDE
BOITIER ERGONOMIQUE POUR SONDE ULTRASONORE A TRANSDUCTEUR

(30) Priority: 04.12.2003 US 526818 P
(43) Date of publication of application: 23.08.2006
(73) Proprietor: Koninklijke Philips Electronics N.V., 5621 BA Eindhoven (NL)
(72) Inventor: YEAGER, Brian, T. SPID Philips Intellectual Property & Standards, 75008 Paris (FR); MCCARDLE, Gyke SPID Philips Intellectual Property & Standards, 75008 PARIS (FR)
(74) Representative: van Oudheusden-Perset, Laure E.
(86) International application number: PCT/IB2004/052623
(87) International publication number: WO 2005/053537

(56) References cited:
- EP-A- 0 439 309
- AU-B2- 674 968
- GB-A- 1 240 969
- GB-A- 2 234 575
- US-A- 4 025 733
- US-A- 5 752 517
- PATENT ABSTRACTS OF JAPAN vol. 1997, no. 06, 30 June 1997 (1997-06-30) & JP 09 033496 A (NOHMI BOSAI LTD), 7 February 1997 (1997-02-07)

## Description

The present invention relates generally to ultrasound transducer probes used in ultrasound imaging systems and more particularly, to with an ultrasound transducer probe with a housing that provides improved ergonomics to the operator of the probe during use.

Ultrasound transducer probes are often used for interoperative imaging of a patient's internal body parts. The probes, also referred to as a ultrasonic or ultrasound scanhead, include a housing and a transducer arranged in the housing in a position such that ultrasound waves are emitted from an active surface of the transducer toward the body part as the housing is passed over the body part. The transducer then receives ultrasound waves reflected from the body part. A cable is connected to the transducer and passing through an opening in the housing to connect the transducer to a control unit and allow for signal transmission between the transducer and the control unit. Signals representative of the received ultrasound waves are provided by the transducer to the control unit via the cable and processed by the control unit to obtain images of the body part.

A drawback in the use of ultrasound probes of this type is that the opening in the housing through which the cable passes is often arranged in a position which does not provide ergonomic use of the probe. For example, in some designs, the cable passes through an opening at the top of the housing of the probe, opposite to the body-contacting surface of the probe, and in a direction perpendicular to the active surface of the probe ("on axis"). Since the probe is held during operation at a position between the body-contacting surface and the top of the housing, and since constant pressure contact is required for suitable imaging, the cable exits the housing from a location above the hand of the operator and the operator is often required to exert a gripping force to securely hold the probe against possibly tension exerted by the cable and the probe/patient contact pressure. Moreover, the operator's wrist may be repeatedly deflected under pressure resulting from the cable's tension and torque.

US 5,752,517 and JP 09-033496 (published on 07-02-1997) disclose prior art ultrasonic probes.

As such, it would be desireable to provide a housing for an ultrasonic transducer which provides improved ergonomics.

It is an object of the present invention to provide a new transducer probe for use in internal ultrasound imaging systems, such as general abdominal and obstetric imaging applications.

It is another object of the present invention to provide a new ultrasonic transducer probe which is designed in consideration of ergonomic factors, e.g., to minimize the hand pressure, gripping force and wrist torque required to be exerted by an operator to operate the probe.

In order to achieve these objects and others, a transducer probe in accordance with the invention includes a housing with an upper portion defining a substantially spherical outer surface adapted to be gripped by an operator of the probe and a lower portion defining a bottom of the housing and a cable exit opening adapted to enable a cable to pass from an interior of the housing to an exterior of the housing. The cable exit opening is thus positioned between the bottom of the housing and the upper portion of the housing. Moreover, a cable conduit extends from the cable exit opening to lead the cable out of the interior of the housing at an angle of about 0° to about 30° relative to a horizontal plane of the housing perpendicular to a central, vertical axis thereof.

The positioning of the cable exit opening below the portion of the housing gripped by the operator, preferably in combination with the orientation of the cable conduit at an angle to the horizontal plane of the housing, is effective to minimize the effective weight/torque which arises during movement of the housing and cable relative to a control unit which is coupled to the cable. During use, the operator's wrist is positioned above the cable exit opening and this has been found to provide a reduction in the impact of tension from the cable and torque being exerted by the cable on the operator's wrist. Furthermore, the substantially spherical outer surface is intended to provide a form suitable for maintaining a "wrist neutral", "natural power grip" hand and wrist position throughout scanning manipulation and to facilitate one-hand rotation of the probe for image plane changes during scanning.

In one embodiment, the housing is made of two, substantially mirror-image components, each having a hemispherical portion which provides half of the upper portion of the housing and a lower arcuate portion which provides half of the lower portion and includes a channel which provides half of the cable conduit. One or both of the housing components may be provided with a mounting structure to enable the transducer to be securely mounted to the housing. The components are provided with a cooperating or complementary attachment structure, e.g., a projection and a recess, to enable them to the secured together.

A transducer probe in accordance with the invention includes the housing in any of the forms described above, a transducer arranged in the interior of the housing and having an active surface oriented toward a bottom of the housing and a cable for connecting the transducer to a control unit of the probe. A lens may be attached to the housing or to the transducer in a position opposite the active surface of the transducer. A loadable, flexible epoxy material is optionally arranged in an interior of the housing and variably loaded to counter balance the cable.

Use of a probe including the housing described above provides improved ergonomic properties which enhance the operator's ease of use of the probe.

The invention, together with further objects and advantages thereof, may best be understood by reference to the following description taken in conjunction with the accompanying drawings, wherein like reference numerals identify like elements and wherein:
FIG. 1 is a perspective view of a housing of an ultrasound transducer probe in accordance with the present invention.
FIG. 2 is an internal view of an ultrasound transducer probe including the housing shown in FIG. 1.
FIG. 3 is a front view of a first component of the housing shown in FIG. 1.
FIG. 4 is a bottom view of the housing component shown in FIG. 3.
FIG. 5 is a cross-sectional view of the housing component shown in FIG. 3 taken along the line 5-5 of FIG. 3.
FIG. 6 is an enlarged view of the section designated A in FIG. 5.
FIG. 7 is a front view of a second component of the housing shown in FIG. 1.
FIG. 8 is a bottom view of the housing component shown in FIG. 7.
FIG. 9 is a cross-sectional view of the housing component shown in FIG. 7 taken along the line 9-9 of FIG. 7.
FIG. 10 is an enlarged view of the section designated B in FIG. 9.

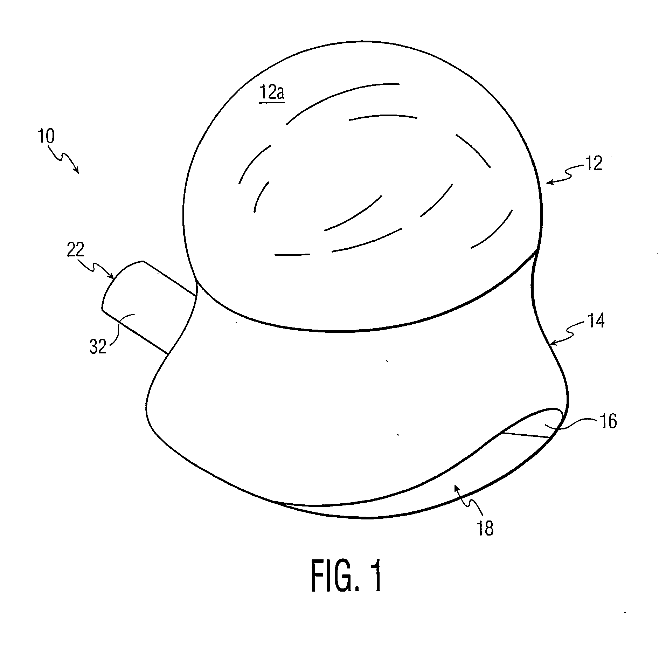

Referring to the accompanying drawings wherein like reference numerals refer to the same or similar elements, FIG. 1 shows a housing for an ultrasonic transducer probe in accordance with the invention which is designated generally as 10. The housing 10 includes an upper portion 12 having a substantially spherical outer surface 12a and a lower portion 14 having the approximate, general shape of a truncated cone. The upper portion 12 is designed such that the operator of the probe can easily place their hand over it. The lower portion 14 defines a bottom of the housing 10 and includes inwardly directed lips 16 defining an opening 18 through which ultrasonic waves are transmitted and received during use of the probe.

One of the objectives of a housing for a transducer probe in accordance with the invention is to facilitate operation of the probe by an operator. To this end, the spherical outer surface 12a of the upper portion 12 is designed to fit the "power grip" or natural position of the human hand and easily facilitate one-handed rotation and angulation of the probe.

Another objective of a housing in accordance with the invention is to provide an exit for a cable, which typically connects a transducer in the housing to a control unit which controls the probe, in a particular position relative to the portion of the housing which is gripped by the operator and which position has been found to provide improved ergonomic use of the probe. Specifically, in the invention, the housing 10 is designed to provide an exit for a cable at a position below the portion of the housing which is gripped by the operator and structure for guiding the cable from the exit in a direction at a narrow angle to a horizontal plane of the housing. This is in contrast to prior art probes wherein the cable exits from a position above the portion of the housing of the probe which is gripped by the operator during use and in a direction perpendicular to the horizontal plane of the probe and perpendicular to the plane in which the operator's wrist is positioned during use of the probe.

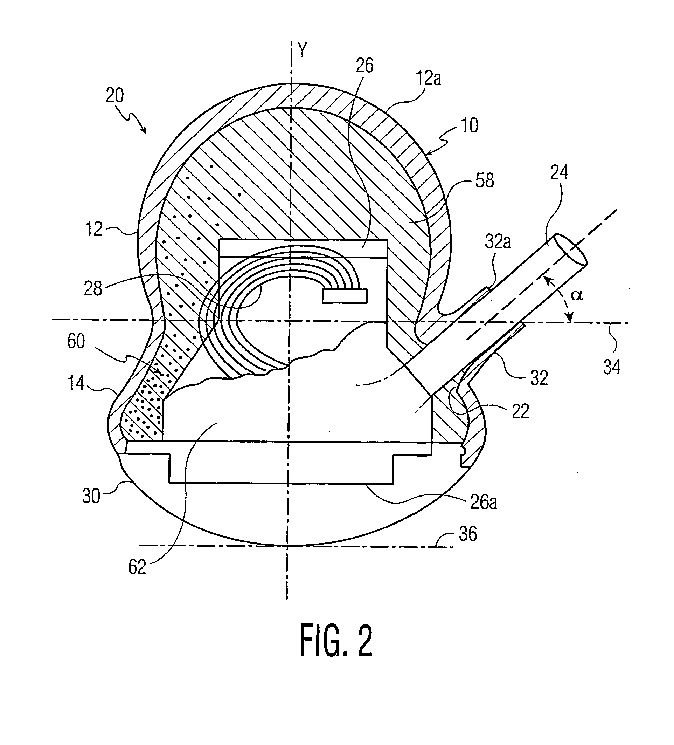

To achieve this objective, reference is made to FIG. 2 which shows an ultrasonic transducer probe 20 including the housing 10 in accordance with the invention. As shown, the lower portion 14 of the housing 10 includes a cable exit opening 22 through which a cable 24 passes to connect the transducer 26 mounted in the housing 10 to the control unit (not shown). Wires 28 of the cable 24 are connected to the transducer 26 which may be any type of transducer capable of performing imaging. Thus, in the housing 10, the cable exit opening 22 is arranged below the upper portion 12 which is gripped by the operator during use of a probe including the housing 10.

The probe 20 also includes a convex lens 30 through which the ultrasonic waves pass to and from the active surface of the transducer 26. Lens 30 may be part of the housing 10 and attached thereto by any means known in the art, such as by an adhesive agent. In the alternative, the lens 30 may be attached to or formed in connection with the transducer 26.

Moreover, to further improve the ergonomics of the housing 10, the housing 10 includes a cable conduit 32 leading from the cable exit opening 22 and which is designed to lead or orient the cable 24 out of the housing 10 in a particular direction. Specifically, the cable conduit 32 leads from the cable exit opening 22 in a direction at an angle to a horizontal plane 34 of the housing 10, horizontal plane 34 being a plane perpendicular to the vertical, central axis Y of the housing 10 (see FIG. 2). This angle is designated α in FIG. 2 and ranges from about 0° to about 30°, an angle of 0° means that the cable conduit 32 leads the cable 24 out of the housing 10 in a direction parallel to the horizontal plane 34 of the housing 10. The transducer 26 is often usually arranged such that its active surface 26a is parallel to the horizontal plane 34 of the housing 10 and thus the cable 24 would exit in a direction at an angle to the active surface 26a of the transducer 26.

The specific angle at which the cable conduit 32 leads the cable 24 from the housing 10 depends in part on the distance between the outer edge 32a of the cable conduit 32 and the body-contacting plane 36 of the probe 20. Generally, if the outer edge 32a of the cable conduit 32 is close to a horizontal body-contacting plane 36 of the probe 20, then the angle α will be higher than if the outer edge 32a is farther from the body-contacting plane 36. This is because a larger angle is needed the lower the outer edge 32a is to the body-contacting plane 36 in order to prevent the cable 22 from draping down onto the patient during examination.

In the probe 20, it is possible to selectively weight the probe 20 to balance it in its neutral position. This can be accomplished by filling a part or all of the available interior of the housing 10 with a loaded, flexible epoxy 58, or similar material, which may be appropriately loaded or weighted depending on the construction of the housing 10 and the cable 24 (see FIG. 2). For example, the loading of the epoxy 58 may be heavier in the front/nose 60 of the housing 10 of the probe 20 (that portion of the housing 10 opposite from the cable exit opening 22) to counterbalance the cable 24 (the heavier loading being represented by the gradually increasing dots toward the front/nose 60 in FIG. 2). This intentional weighting of the interior of the probe 20 is effective to counterbalance residual torque caused by the cable 24, which may occur in spite of the specific positioning of the cable exit 32 as described above.

An EMI-RFI shield layer 62 may also be provided around the transducer 26.

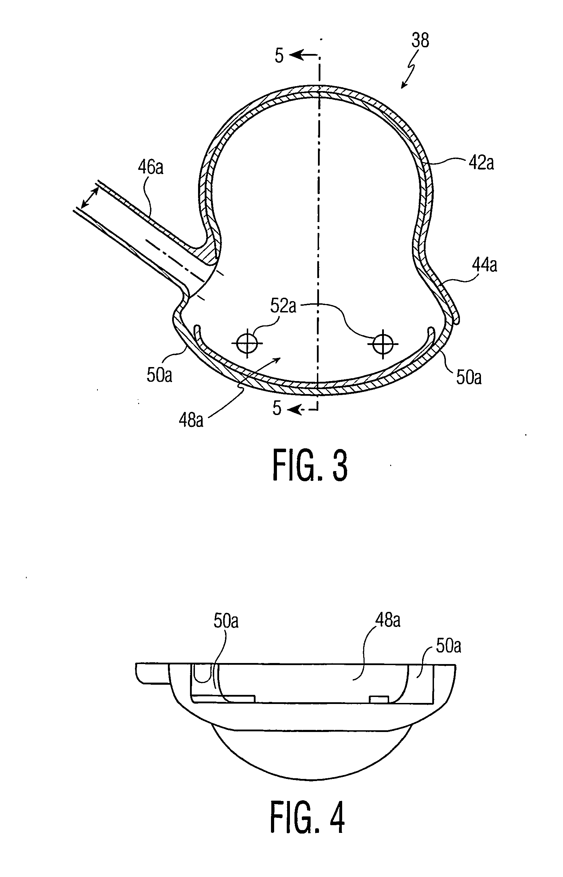

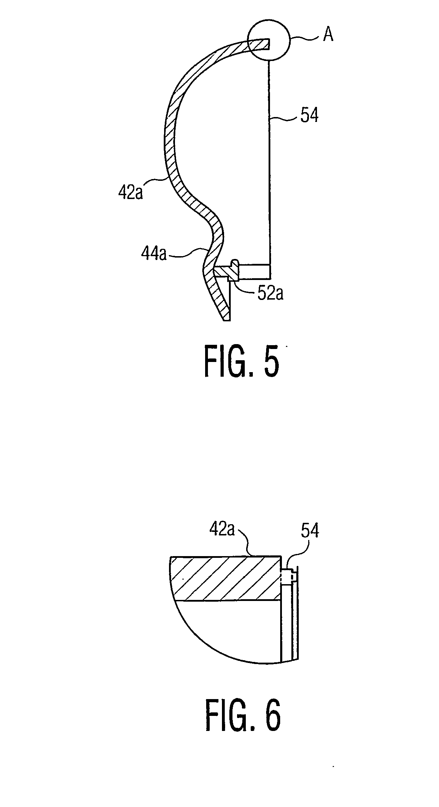

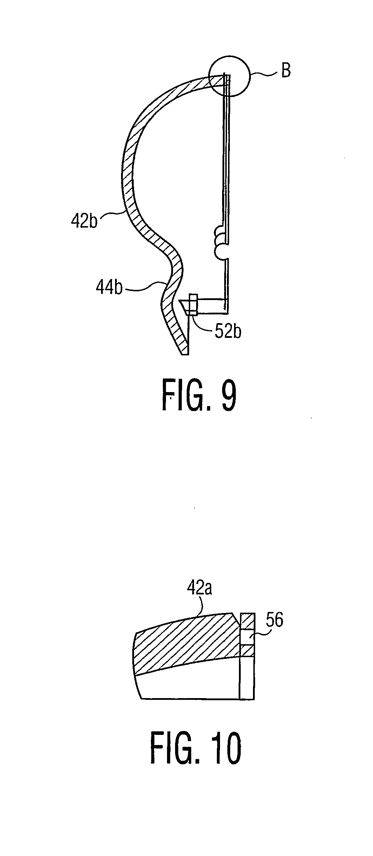

Referring now to FIGS. 3-10, housing 10 may be made of two components 38,40, which are substantially mirror-image components (with some differences as described below). Each housing component 38,40 may be made of molded plastic or similar material(s). Although the illustrated embodiment shows the fabrication of the housing 10 from two separate components which are attached together to form the complete housing 10, it is possible and conceivable to form the housing 10 from a unitary member and this form of construction is also intended to be within the scope of the invention.

The first housing component 38 is shown in FIGS. 3-6 and has a hemispherical portion 42a which provides half of the upper portion 12 of the housing 10 and a lower arcuate portion 44a which provides half of the lower portion 14 and includes a channel 46a which provides half of the cable conduit 32. A recess 48a is defined by the inwardly directed flanges 50a which corresponds to half of the opening 18 defined by the lips 16. One or more mounting mechanisms 52a are formed on the inner surface of the component 38 to enable the transducer 26 and/or other internal probe electronics to be mounted thereto. To enable connection of the housing components 38, 40 together, the housing component 38 includes a projection 54 extending from a lateral surface defining an edge of the housing component 38, and the projection 54 is designed to mate with a corresponding recess 56 formed in a lateral surface defining an edge of the housing component 40.

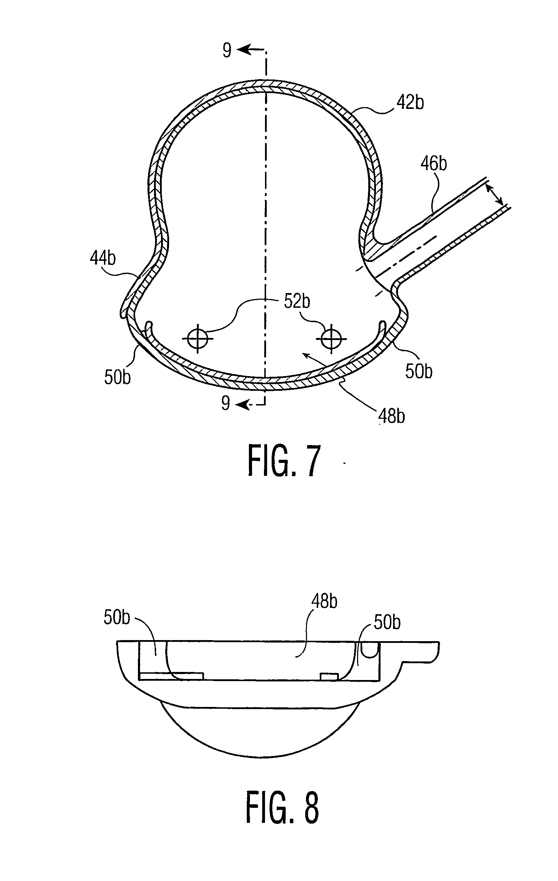

The second housing component 40 is shown in FIGS. 7-10 and has a hemispherical portion 42b which provides half of the upper portion 12 of the housing 10 and a lower arcuate portion 44b which provides half of the lower portion 14 and includes a channel 46b which provides half of the cable conduit 32. A recess 48b is defined by the inwardly directed flanges 50b which corresponds to half of the opening 18 defined by the lips 16. Instead of or in addition to the mounting mechanisms 52a formed on the inner surface of the first housing component 38, one or more mounting mechanisms 52b may be formed on the inner surface of the second housing component 40 to enable the transducer 26 and/or other internal probe electronics to be mounted thereto. As noted above, a recess 56 is formed in a lateral surface defining an edge of the housing component and accommodates the projection 54 extending from the lateral surface defining the edge of the first housing component. Other mechanisms for attaching the first and second housing components 38,40 together can also be used in the invention.

To assemble the probe 20, the electronics to be placed in the interior of the housing 10, e.g., the transducer 26, are connected to wires 28 of a cable 24, and mounted to one of the housing components, e.g., the first housing component 38, via the respective mounting mechanism 52a and such that the cable 24 passes through the channel 46a. The second housing component 40 is then attached to the first housing component 38 having the transducer 26 mounted thereto via the cooperating projection 54 and recess 56 in the lateral edges of the housing components 38,40. At the same time, the mounting mechanism 52b in the second housing component 40 may be coupled to the electronics to thereby secure the transducer 26 within the housing 10. If the lens 30 is part of the housing 10, then it is first attached to one of the housing 38, 40 and then to the other housing component 38, 40 when the housing components 38, 40 are attached to one another. If the lens 30 is part of the transducer 26, then it is mounted to the first housing component 38 and adhered thereto and then when the second housing component 40 is attached to the first housing component 38, it is attached thereto.

In view of the positioning of the cable exit opening 22 below the upper portion 12 of the housing 10 which is gripped by the operator and orienting it at a non-perpendicular angle to the horizontal plane 34 of the housing 10, the effective contribution of weight/torque by the cable 24 during use of the probe 20 is minimized. An effectively ergonomic housing for a transducer probe is therefore provided.
Although illustrative embodiments of the present invention have been described herein with reference to the accompanying drawings, it is to be understood that the invention is not limited to these precise embodiments, and that various other changes and modifications may be effected therein by one of ordinary skill in the art without departing from the scope of the invention.

## Claims

1. A transducer probe (20), comprising:
a housing (10) defining an interior;
an ultrasonic transducer (26) arranged in said interior of said housing and having an active surface oriented toward a bottom of said housing; and
a cable (24) for connecting said transducer to a control unit of the probe,
said housing (10) including an upper portion (12) substantially shaped according to a sphere and defining a substantially spherical outer surface having the diameter of said sphere and being adapted to be gripped by an operator of the probe and said housing further including a lower portion (14) defining a bottom of the housing and a cable exit opening (22) through which said cable passes from an interior of said housing to an exterior of said housing, said cable exit opening being positioned between the bottom of said housing and said upper portion.

2. The probe of claim 1, wherein said lower portion includes a cable conduit (32) extending from said cable exit opening (22) and leading said cable out of said interior of said housing at an angle of about 0° to about 30° relative to a horizontal plane (34) of said housing perpendicular to a central, vertical axis of said housing.

3. The probe of claim 2, wherein said housing comprises first and second housing components (38, 40), each of said first and second housing components providing approximately half of said upper portion and half of said lower portion.

4. The probe of claim 3, wherein each of said first and second housing components has a hemispherical portion (42b) which provides half of said upper portion of said housing and a lower arcuate portion (44a) which provides half of said lower portion and includes a channel (46a) which provides half of said cable conduit (32).

5. The probe of claim 3, wherein at least one of said first and second housing components includes a mounting mechanism (52a) for enabling mounting of said transducer in said housing.

6. The probe of claim 3, wherein said first and second housing components comprise attachment means for enabling attachment of said first and second housing components together.

7. The probe of claim 6, wherein said attachment means comprises a projection arranged on said first housing component and a corresponding recess formed on said second housing component.

8. The probe of claim 1, wherein said upper portion is substantially spherical.

9. The probe of claim. 1, further comprising a loadable, flexible epoxy material arranged in an interior of said housing, said epoxy material being variably loaded to counter balance said cable.

10. The probe of claim 1, wherein said lower portion defines a opening through which ultrasonic waves are transmitted and received by said ultrasonic transducer during use of the probe.

11. The probe of claim 10, further comprising a lens (30) arranged at the bottom of said housing.

12. The probe of claim 1, wherein said lower portion has the approximate shape of truncated cone.

## Patentansprüche

1. Wandlersonde (20) mit:
einem Gehäuse (10), das einen Innenraum definiert;
einem Ultraschallwandler (26), der im genannten Innenraum des genannten Gehäuses angeordnet ist und dessen aktive Oberfläche auf einen Boden des genannten Gehäuses ausgerichtet ist; und
einem Kabel (24) zum Verbinden des genannten Wandlers mit einer Steuereinheit der Sonde,
wobei das genannte Gehäuse (10) einen oberen Teil (12) hat, der im Wesentlichen entsprechend einer Kugel geformt ist und eine im Wesentlichen sphärische Außenfläche mit dem Durchmesser der genannten Kugel definiert und dafür ausgelegt ist, von einem Bediener der Sonde gegriffen zu werden, und wobei das genannte Gehäuse weiterhin einen unteren Teil (14) umfasst, der einen Boden des Gehäuses und eine Kabelaustrittsöffnung (22) definiert, durch die das genannte Kabel vom Innenraum des genannten Gehäuses zu einem Äußeren des genannten Gehäuses geführt wird, wobei die genannte Kabelaustrittsöffnung zwischen dem Boden des genannten Gehäuses und dem genannten oberen Teil angeordnet ist.

2. Sonde nach Anspruch 1, wobei der genannte untere Teil einen Kabelkanal (32) umfasst, der von der genannten Kabelaustrittsöffnung (22) ausgeht und das genannte Kabel aus dem genannten Innenraum des genannten Gehäuses in einem Winkel von ca. 0° bis ca. 30° zu einer horizontalen Ebene (34) des genannten Gehäuses senkrecht zu einer zentralen, vertikalen Achse des genannten Gehäuses herausführt.

3. Sonde nach Anspruch 2, wobei das genannte Gehäuse erste und zweite Gehäusekomponenten (38, 40) umfasst, wobei sowohl die erste als auch die zweite genannte Gehäusekomponente jeweils ca. die Hälfte des genannten oberen Teils und die Hälfte des genannten unteren Teils schaffen.

4. Sonde nach Anspruch 3, wobei sowohl die genannte erste als auch die genannte zweite Gehäusekomponente einen hemisphärischen Teil (42b) haben, der die Hälfte des genannten oberen Teils des genannten Gehäuses schafft, und einen unteren gewölbten Teil (44b), der die Hälfte des genannten unteren Teils schafft und einen Kanal (46a) enthält, der die Hälfte des genannten Kabelkanals (32) schafft.

5. Sonde nach Anspruch 3, wobei mindestens entweder die erste oder die zweite Gehäusekomponente einen Montagemechanismus (52a) enthält, um die Montage des genannten Wandlers in dem genannten Gehäuse zu ermöglichen.

6. Sonde nach Anspruch 3, wobei die genannte erste und die genannte zweite Gehäusekomponente Befestigungsmittel umfassen, um die Befestigung der genannten ersten und der genannten zweiten Gehäusekomponente aneinander zu ermöglichen.

7. Sonde nach Anspruch 6, wobei das genannte Befestigungsmittel einen Vorsprung hat, der an der genannten ersten Gehäusekomponente vorgesehen ist, und eine entsprechende Aussparung, die an der genannten zweiten Gehäusekomponente gebildet ist.

8. Sonde nach Anspruch 1, wobei der genannte obere Teil im Wesentlichen sphärisch ist.

9. Sonde nach Anspruch 1, weiterhin mit einem belastbaren, flexiblen Epoxidmaterial, das in einem Innenraum des genannten Gehäuses angeordnet ist, wobei das genannte Epoxidmaterial variabel belastet wird, um das genannte Kabel zu kompensieren.

10. Sonde nach Anspruch 1, wobei der genannte untere Teil eine Öffnung definiert, durch die Ultraschallwellen von dem genannten Ultraschallwandler während des Betriebs der Sonde ausgesendet und empfangen werden.

11. Sonde nach Anspruch 10, weiterhin mit einer Linse (30), die am Boden des genannten Gehäuses angeordnet ist.

12. Sonde nach Anspruch 1, wobei der genannte untere Teil ungefähr die Form eines Kegelstumpfs hat.

## Revendications

1. Sonde de capteur (20) comprenant:
un boîtier (10) qui définit un intérieur,
un capteur ultrasonique (26) qui est disposé dans ledit intérieur dudit boîtier et qui présente une surface active étant orientée vers un fond du boîtier; et
un câble pour connecter ledit capteur à une unité de commande de la sonde,
ledit boîtier (10) comprenant une partie supérieure (12) qui est sensiblement mise en forme selon une sphère et qui définit une surface extérieure sensiblement sphérique ayant le diamètre de ladite sphère et qui est adaptée de manière à être saisie par un opérateur de la sonde, et ledit boîtier comprenant encore une partie inférieure (14) qui définit un fond du boîtier et une ouverture de sortie de câble (22) à travers laquelle ledit câble passe à partir d'un intérieur dudit boîtier à un extérieur dudit boîtier, ladite ouverture de sortie de câble étant positionnée entre le fond dudit boîtier et ladite partie supérieure.

2. Sonde selon la revendication 1, dans laquelle ladite partie inférieure comprend un conduit de câble (32) qui s'étend à partir de ladite ouverture de sortie de câble (22) et qui mène ledit câble hors dudit intérieur dudit boîtier sous un angle de l'ordre de 0° jusqu'à environ 30° par rapport à un plan horizontal (34) dudit boîtier étant perpendiculaire à un axe central et vertical dudit boîtier.

3. Sonde selon la revendication 2, dans laquelle ledit boîtier comprend des premier et deuxième composants de boîtier (38, 40), chacun desdits premier et deuxième composants de boîtier fournissant environ la moitié de ladite partie supérieure et la moitié de ladite partie inférieure.

4. Sonde selon la revendication 3, dans laquelle chacun desdits premier et deuxième composants de boîtier présente une partie hémisphérique (42b) qui fournit la moitié de ladite partie supérieure dudit boîtier et une partie arquée inférieure (44a) qui fournit la moitié de ladite partie inférieure, et comprend un canal (46a) qui fournit la moitié dudit conduit de câble (32).

5. Sonde selon la revendication 3, dans laquelle au moins un desdits premier et deuxième composants de boîtier comprend un mécanisme de montage (52a) qui permet le montage dudit capteur dans ledit boîtier.

6. Sonde selon la revendication 3, dans laquelle lesdits premier et deuxième composants de boîtier comprennent des moyens de fixation pour permettre la fixation desdits premier et deuxième composants de boîtier l'un à l'autre.

7. Sonde selon la revendication 6, dans laquelle lesdits moyens de fixation comprennent une saillie qui est agencée sur ledit premier composant de boîtier et un évidement correspondant qui est pratiqué sur ledit deuxième composant de boîtier.

8. Sonde selon la revendication 1, dans laquelle ladite partie supérieure est sensiblement sphérique.

9. Sonde selon la revendication 1, comprenant encore une matière époxyde chargeable et flexible qui est agencée dans un intérieur dudit boîtier, ladite matière époxyde étant chargée variablement pour contrebalancer ledit câble.

10. Sonde selon la revendication 1, dans laquelle ladite partie inférieure définit une ouverture à travers laquelle des ondes ultrasoniques sont transmises et sont reçues par ledit capteur ultrasonique pendant l'utilisation de la sonde.

11. Sonde selon la revendication 10, comprenant encore un objectif (30) qui est disposé au fond dudit boîtier.

12. Sonde selon la revendication 1, dans laquelle ladite partie inférieure présente la forme approximative d'un cône tronqué.
